(19) Europäisches Patentamt
European Patent Office
Office européen des brevets

(11) EP 1 886 699 B1

(12) **EUROPEAN PATENT SPECIFICATION**

(45) Date of publication and mention
of the grant of the patent:
**25.04.2012 Bulletin 2012/17**

(51) Int Cl.:
***A61L 15/60*** *(2006.01)*      ***A61L 15/18*** *(2006.01)*

(21) Application number: **06118515.3**

(22) Date of filing: **07.08.2006**

(54) **Absorbent articles comprising superabsorbent polymers and a material with radiation-induced hydrophilicity**

Absorbierende Artikel, die superabsorbierende Polymere und ein strahleninduziert hydrophiles Material enthalten

Articles absorbants comprenant des polymères superabsorbants et un matériau ayant une hydrophilicité induite par radiation

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**13.02.2008 Bulletin 2008/07**

(73) Proprietor: **The Procter & Gamble Company Cincinnati, OH 45202 (US)**

(72) Inventor: **Grota, Juliane**
**65824, Schwalbach (DE)**

(74) Representative: **Heide, Ute et al**
**Procter & Gamble Service GmbH**
**Sulzbacher Strasse 40-50**
**65824 Schwalbach am Taunus (DE)**

(56) References cited:
EP-A- 1 669 394          EP-A1- 1 498 543
EP-A2- 0 287 970          WO-A1-02/083193
US-A- 5 244 934          US-A- 5 419 956
US-A1- 2004 033 270

• MASAHIRO MIYAUCHI, AKIRA NAKAJIMA, TOSHIYA WATANABE AND KAZUHITO HASHIMOTO: "Photocatalysis and Photoinduced Hydrophilicity of Various Metal Oxide Thin Films" CHEM. MATER., 2002, XP002420776

**Description**

FIELD OF THE INVENTION

This invention relates to an absorbent article comprising superabsorbent material comprising superabsorbent polymers that are mixed or coated with a surface-modifying material that renders the superabsorbent material highly hydrophilic, whereby the surface modifying agent is preferably a material that has (and/or provides) radiation-induced hydrophilicity.
The invention also relates to a process of making such superabsorbent material involving either combining the super absorbent polymers and said material that has radiation-induced hydrophilicity; and/ or combining the superabsorbent polymers and a material with radiation-inducible hydrophilicity, which is subsequently submitted to the appropriate radiation.

BACKGROUND OF THE INVENTION

An important component of disposable absorbent articles such as diapers are absorbent cores with superabsorbent polymers (SAP), also referred to as superabsorbent polymers superabsorbers, absorbent gelling material, AGM, or hydrogel-forming polymers. This polymeric material ensures that large amounts of bodily fluids, e.g. urine, menses, can be absorbed by the article during its use and locked away, thus providing low rewet and good skin dryness. At relatively high concentrations, an important property of these superabsorbent polymers is their permeability/flow conductivity. The permeability/flow conductivity of a material can be defined in terms of its Saline Flow Conductivity (SFC). SFC is a measure of the ability of a material to be permeable (even under a certain pressure) for saline fluid flow, such as the ability of a layer comprising swollen absorbent polymer to be permeable for body fluid flow. Absorbent polymers with relatively high permeability (SFC values) can be made by increasing the level of crosslinking, which increases the strength of the swollen gel, butthis typicallyalso reduces the absorbent capacity of the gel undesirably. One key focus to increase the SFC of superabsorbent material so far has been to modify the surface of the SAP via cross-linking such that an optimum SFC value and absorbent capacity are achieved at the same time, e.g. such that one does not compromise the other too much.
In addition to surface crosslinking, several other approaches have been utilized to improve the permeability, and/or the absorption rate of the absorbent core. For example, U.S. Patent 5,419,956 (Roe), issued on May 30, 1995, describes the addition of inorganic particles such as silica to superabsorbent polymers to improve fluid uptake rate and to increase fluid distribution. GB-A-2.082.614 (Richman) describes the addition (either under wet or dry conditions) of between 1 and 75% of an extender material such as cellulose derivatives, or inorganic materials like clay or minerals.

EP1669394 describes an absorbent article comprising a superabsorbent material, comprising superabsorbent polymers and a material, wherein said mixture are submitted to UV irradiation.

EP20287970 refers to a process for producing a highly water absorptive polymer and the corresponding absorbent article comprising superabsorbent polymers => no semiconductor material nor submission to radiation.

US5244934 describes a process for producing a highly water absorptive polymer and corresponding absorbent article by UV irradiation => no semiconductor material nor submission to radiation.

US2004/0033270 relates to hygiene product such as diapers, tampons, pantyliners comprising an absorbent article and zinc oxide (ZnO) in the form of nanoparticles => no radiation suitable to induce hydrophilicity.

WO02/083193 discloses a multifunctional menstrual pad comprising an absorbent core and a bioceramic substance comprised of $SiO_2$, $Al_2O_3$, $Fe_2O_3$ and $TiO_2$ in the pulp fluff => no radiation suitable to induce hydrophilicity.

EP1498543 discloses an absorbent article comprising suitable material to reduce odour such as zinc oxide (ZnO), calcium oxide, magnesium oxide, etc => no radiation suitable to induce hydrophilicity.

However, there is still a need to improve the permeability of absorbent articles or cores, and/ or to improve the absorbent capacity and gel strength at the same time.
Also there is still a need to provide even thinner absorbent articles with high SAP concentrations which, despite the high concentration of the SAP's, do not significantly suffer from gel blocking. There is also a need to provide absorbent cores with improved fluid distribution.
Furthermore, the inventor found that there is a further need for absorbent articles with an improved wettabillity. It was found that there is a need to provide absorbent articles with superabsorbent material that, in addition to the desired swelling behaviors of the superabsorbent material, (such as sorption capacity, permeability, sorption under load and swell speeds, including preferred high SFC and CRC values), has an improved wettability with aqueous liquids or water-containing liquids, such as urine and blood, resulting in an improved liquid transport via improved capillary pressure.

The inventor has now found an improved, highly hydrophilic superabsorbent material with improved wettability that may provide improved liquid transport or distribution, and that can be used in absorbent articles at high concentrations and/ or densities and for example even in fiber-free absorbent core structures.

The inventor has also found that a preferred route to provide highly hydrophilic superabsorbent material involves combining (preferably partially or completely coating) the superabsorbent polymers with a, preferably particulate, material with radiation-induced hydrophilicity, such as UV-induced metal oxides like $TiO_2$, $SnO_2$, $ZnO$, $WO_3$, $V_2O_5$.

SUMMARY OF THE INVENTION

The present invention relates to an absorbent article comprising a superabsorbent material, comprising superabsorbent polymers and a semiconductor material with radiation-induced hydrophilicity, obtainable by

a) combining said superabsorbent polymers and said material with radiation-induced hydrophilicity; and/ or
b) combining said superabsorbent polymers and a material with radiation-inducible hydrophilicity, to obtain a mixture and submitting said mixture to radiation suitable to induce said hydrophilicity.

The invention also relates to an absorbent article comprising superabsorbent material comprising superabsorbent polymers having a Fluid Rise Height Ratio (FRH ratio) of at least 1.10. Said superabsorbent material may thereto preferably comprise a surface-modifying material. Said surface-modifying material may be a material with radiation-induced hydrophilicity.

DETAILED DESCRIPTION

Absorbent articles

The absorbent article of the invention may be any absorbent article used to absorb and retain liquids, such as urine, blood. The absorbent article is typically a disposable absorbent article to be worn against the skin, such as preferably interlabial products, sanitary napkins, panty liners, adult incontinent products, baby or infant diapers, and training pants.
The absorbent article of the invention comprises the superabsorbent material herein in an absorbent structure, including for example an acquisition layer, distribution layer, and/ or in a storage layer, e.g. preferably in (at least) the storage layer of an absorbent article. In other words, the superabsorbent material in an absorbent structure may at the same time serve to acquire, distribute and/ or store the bodily fluid, but typically it will at least serve for fluid storage. The absorbent article may also comprise two or more absorbent structures or layers that comprise the superabsorbent material herein, or two or more absorbent structures or layers that comprise chemically different superabsorbent material herein. The absorbent article herein may also comprise the superabsorbent material described herein and superabsorbent polymers not comprising the material with radiation-induced hydrophilicity.
The absorbent article herein may comprise an absorbent structure that contains one or more regions comprising the superabsorbent material herein, and that contains one or more regions comprising a second, different superabsorbent material, or comprising no superabsorbent material. A "region", when used herein, is a part of an absorbent structure with a surface area of at least 20 mm x 15 mm (length x width, typically machine direction x cross-machine direction). The former may be obtained by providing an absorbent structure with superabsorbent material mixed with the material with radiation-inducible hydrophilicity, as described herein, and only selectively activating said material in certain region (s) but not in other regions within said absorbent structure or layer, so obtain a structure or layer with regions with superabsorbent material with radiation-induced hydrophilicity and regions with superabsorbent material without radiation-induced hydrophilicity.
The absorbent article may comprise the superabsorbent material herein at any weight level or concentration. The absorbent article may comprise one or more absorbent structures or layers of the superabsorbent material herein, mixed with absorbent cellulose fibers, such as pulp, or modified absorbent cellulose fibers. However, in one embodiment, the absorbent article comprises a storage layer comprising large amounts of the superabsorbent material herein compared to any other components, like absorbent cellulose fibers. For example, the ratio of superabsorbent material to other components in the absorbent structure may be from 100:1 1 to 2:1, preferably from 100:1 to 3:1 or more preferably to 9:1 or even to 20:1. It may be preferred that the absorbent structure is substantially free from cellulose absorbent fibers, e.g. that it comprises less than 5% by weight of the superabsorbent material of such cellulose fibers, or preferably even less than 3% by weight, or even less than 1 % by weight.
The absorbent article may as alternative to the absorbent cellulose fibers, or in addition thereto, comprise a structuring agent or matrix agent, such as a thermoplastic material, and/ or an adhesive material, for example a fibrous thermoplastic and/ or adhesive material, applied under, above or mixed with the superabsorbent material in said absorbent structure. Such structuring agents or matrix agents may for example be present at a weight level of up to 40% by weight of the superabsorbent material, but preferred are lower levels of for example from 0.05% or 0.1% or 0.5% to 20%, 10% or 5% by weight.

The absorbent structure, e.g. acquisition/ distribution and/ or storage layer(s), of the absorbent article comprising the superabsorbent material herein may comprise one or more core cover sheets, or a core wrap, as known in the art. The core cover layer or layers may for example comprise at least one sheet of a nonwoven material. One preferred material is a so-called SMS material, comprising a spunbonded, a melt-blown and a further spunbonded layer. Highly preferred are permanently hydrophilic nonwovens, and in particular nonwovens with durably hydrophilic coatings. An alternative preferred material comprises a SMMS-structure. The top layer and the bottom layer may be provided from two or more separate sheets of materials or they may be alternatively provided from a unitary sheet of material. Preferred nonwoven materials are provided from synthetic fibers, such as PE, PET and most preferably PP. As the polymers used for nonwoven production are inherently hydrophobic, they are preferably coated with hydrophilic coatings, e.g. coated with nanoparticles, as known in the art.

The absorbent article herein may also comprise one or more pockets of the superabsorbent material herein, which may be removable and/ or replaceable pockets.

The absorbent article may also comprise one or more layers of the superabsorbent material herein that are non-uniform or discontinuous.

Preferred disposable absorbent article herein are sanitary napkins, panty liners, adult incontinence products and infant diapers or training or pull-on pants, whereby articles which serve to absorb urine, e.g. adult incontinence products, diapers and training or pull-on pants are most preferred articles herein. Preferred absorbent articles herein have a topsheet, facing the skin of the wearer, and a backsheet, which each have a front region, back region and crotch region, positioned therein between. The absorbent structure of the invention is typically positioned in between the topsheet and backsheet. The backsheets may be (moisture-) vapour pervious but liquid impervious. The topsheet material is at least partially liquid permeable; it may comprise one or more large opening, or a multitude of apertures, so-called apertured (e.g. formed film) topsheets. They may alternatively or in addition be hydrophilic. Preferred may be that the topsheet comprises a skin care composition, e.g. a lotion. The sanitary napkins or panty liners typically comprise a fastening means on the backsheet, to fasten the article to the under wear of the user. The diapers herein have preferably front and rear waist bands, comprising a fastening system, to fasten the front waist band to the rear waist band, typically comprising one or more fastening member, being part of or attached to the rear waist band, and one ore more landing members, being part of or attached to the front waist band. Training pants herein may have a pre-fastened waist band that may in addition comprise fastening means to re-fastening the training pants during use.

The adult incontinence products, diapers and training pants herein may have one or more sets of leg cuffs and/ or barrier cuffs. It may also be that the topsheet has one or more openings, preferably with elastication means along the length thereof, where through waste material can pass into a void space above the absorbent structure, and which ensures it is isolated in this void space, away from the wearer's skin.

The absorbent structure(s) of the absorbent article, or region thereof, comprising the superabsorbent material herein may be very thin, having an average caliper of 3 mm or less, or for example 2 mm or less.

The absorbent structure (s) comprising the superabsorbent material herein may have one or more regions (of 20 mm x 15 mm) that may have a basis weight of superabsorbent material of 100 $g/m^2$ or more, or for example 250 g/ $m^2$ or more.

The absorbent structure comprising the superabsorbent material may have one or more regions that may have a density of 0.1g/ $cm^3$ or more, preferably 0.15 $g/cm^3$ or more, or preferably 0.20 $g/cm^3$ or more, or preferably 0.3 g/ $cm^3$ or more.

In one embodiment, the absorbent article comprises one or more absorbent structures or layers with one or more regions comprising the superabsorbent material, whereby said regions(s) have a basis capacity of more than 3 $l/m^2$, preferably of more than 5 $l/m^2$, or preferably of more than 6.5 l/ $m^2$, or for example more than 5 l/ $m^2$, or more than 6.5 $l/m^2$, or more than 8 $l/m^2$ or in some embodiments, more than 10 $l/m^2$, or preferably more than 12 $l/m^2$, as can be determined by the method described herein.

In addition, or as alternative, the absorbent article may comprises one or more absorbent structures or layer with one or more regions comprising the superabsorbent material described herein, whereby said region(s) have an absorption efficiency of at least 2 ml/ $cm^3$, or preferably at least 3 ml/ $cm^3$, or preferably at least 5 $ml/cm^3$, or in some embodiments at least 6.5 $ml/cm^3$, or at least 8 $ml/cm^3$, as can be determined by the method described below.

In addition, or as alternative, the absorbent article may comprise one or more absorbent structures with region (s) in the first, back 25% part of the absorbent structure (i.e. first 25% part of the longitudinal length of the total absorbent structure, starting from the back transverse edge of the absorbent structure to 25% of the length of the absorbent structure; the "back: being in use closest to the back of the user) comprising the superabsorbent material described herein, whereby said first region(s) in the first back 25% part of the absorbent structure have an absorption efficiency of at least 60%, or more preferably at least 70 % of the absorption efficiency of one or more region(s) in the third 25% part of the absorbent core (i.e. starting at 50% of the total absorbent structure length to 75% of total absorbent structure length), whilst the region(s) in the first and third 25% parts of the absorbent structure have a SFC of at least 30 units, preferably at least 50 units or possibly at least 80 units.

Superabsorbent material

The superabsorbent material herein is capable of absorbing liquids such as urine or blood and it swells thereby, often forming a gel. The superabsorbent material herein comprises superabsorbent polymers, as described below, and material with radiation-induced hydrophilicity that renders the superabsorbent polymers and said material more hydrophilic (compared to a mixture of the same superabsorbent polymers and non-induced material)

The superabsorbent material may comprise a mixture of said superabsorbent polymers and said material with radiation-induced hydrophilicity, typically a homogeneous mixture, or the superabsorbent polymers may be coated with the material with radiation-induced hydrophilicity.

In one embodiment herein, or in a preferred embodiment herein, the superabsorbent material has a FRH ratio of 1.10 or more, or preferably 1.25 or more, or preferably 1.40 or more.

It may be preferred that said FRH ratio is not more than 2.50, or for example not more than 2.40 or not more than 2.30. It may be preferred that the rise in height of n-heptane is higher than 4 cm, or more preferably higher than 5 cm, or more preferably higher than 7 cm, or more preferably higher than 10 cm, or more preferably higher than 12 cm. It may be preferred that the rise in height of n-heptane is not higher than 24 cm, or more preferably not higher than 19 cm.

The superabsorbent material comprises preferably said superabsorbent polymers in particulate form, as described below. Also said material with radiation-induced hydrophilicity may preferably be in particulate form.

The material with radiation-induced hydrophilicity is preferably present at a level of 0.005% to 10% by weight of the superabsorbent material, more preferably from 0.01% to 5% by weight or even from 0.01 % to 2% by weight, or more preferably from 0.01 % to 1 % by weight.

The superabsorbent material herein may also comprise other components, such as fillers, flowing aids, process aids, anti-caking agents, odour control agents, colouring agents, etc.

The superabsorbent material is obtainable by the process described herein, which is preferably such that the resulting material is solid; this includes granules, beads, flakes, fibres, powders, platelets, spheres and other forms known in the art for superabsorbent polymers described herein. Preferably, the superabsorbent material is in the form of particles having a mass median particle size between 10$\mu$m and 2mm, or even between 50 microns and 1mm, or preferably between 100$\mu$m and 800$\mu$m , as can for example be measured by the method set out in for example EP-A-0691133. In one embodiment herein the superabsorbent material herein is in the form of (free flowing) particles with particle sizes between 10 $\mu$m and 1200 $\mu$m or even between 50 $\mu$m and 850 $\mu$m and a mass median particle size between 100$\mu$m and 800 $\mu$m or even 600$\mu$m. In addition, or in another embodiment herein, the superabsorbent material comprises particles that are essentially spherical. In yet another preferred embodiment herein the superabsorbent material herein has a relatively narrow range of particle sizes with the majority (e.g. at least 80% or preferably at least 90% or even at least 95%) of particles having a particle size between 50$\mu$m and 850$\mu$m, preferably between 100$\mu$m and 700$\mu$m, and more preferably between 200$\mu$m and 500$\mu$m.

The superabsorbent material herein preferably comprises less than 10% by weight of residual water content or moisture, or even less than 5% or in one embodiment less than 3% by weight of moisture, or preferably less than 2% by weight of moisture, or preferably less than 1% by weight, and it may be moisture- or water-free. The level of residual water or moisture can be determined by the method described herein below.

Especially preferred superabsorbent materials made by the process herein have a high sorption capacity measured by the Centrifugation Retention Capacity (CRC test) outlined below. In one embodiment, the superabsorbent material herein has a CRC of at least 15 g/g, more preferably at least 18 g/g, even more preferably at least 21 g/g, or at least 25 g/g, or at least 27 g/g and or at least 29 g/g.

Especially preferred superabsorbent materials made by the process herein have a high permeability for liquid such as can be measured by the SFC test disclosed in US 5,599,335, US 5,562,646 and US 5,669,894. Preferably, the superabsorbent material herein has a SFC of more than 30 units, or more preferably more than 50 units, or even more preferably more than 80 units, or even more preferably more than 100 units.

Superabsorbent polymers

The superabsorbent polymers in the absorbent articles herein are preferably solid, preferably in the form of particles, flakes, fibres, agglomerated particles, as described above; most preferably, the superabsorbent polymers are particles having a mass median particle size as specified above for the superabsorbent material.

As used herein, the term "superabsorbent polymer" refers to a polymer which is substantially water-insoluble, superabsorbent and preferably water-gelling, forming a hydrogel, and which has typically a Centrifuge Retention Capacity (CRC) as defined below of at least 10 g/g. These polymers are often also referred to in the art as (super-) absorbent polymers (SAP) or absorbent gelling materials (AGM).

These polymers are typically (lightly) crosslinked polymers, preferably lightly crosslinked hydrophilic polymers. Especially preferred are acid polymers, which contain a multiplicity of acid functional groups such as carboxylic acid groups, or their salts, preferably sodium salts. Examples of acid polymers suitable for use herein include those which are prepared from polymerizable, acid-containing monomers, or monomers containing functional groups which can be converted to acid groups after polymerization. Such monomers include typically olefinically unsaturated carboxylic acids and anhydrides, and mixtures thereof. Some non-acid monomers can also be included, usually in minor amounts, in preparing the absorbent polymers herein. Such non-acid monomers can include, for example, monomers containing the following types of functional groups: carboxylate or sulfonate esters, hydroxyl groups, amide-groups, amino groups, nitrile groups, quaternary ammonium salt groups, and aryl groups (e.g., phenyl groups, such as those derived from styrene monomers. These non-acid monomers are well-known materials and are described in greater detail, for example, in U.S. Patent 4,076,663 (Masuda et al.), issued February 28, 1978, and in U.S. Patent 4,062,817 (Westerman), issued December 13, 1977.

Olefinically unsaturated carboxylic acid and anhydride monomers useful herein include the acrylic acids typified by acrylic acid itself, methacrylic acid, $\alpha$-chloroacrylic acid, $\alpha$-cyanoacrylic acid, $\beta$-methylacrylic acid (crotonic acid), $\alpha$-phenylacrylic acid, $\beta$-acryloxypropionic acid, sorbic acid, $\alpha$-chlorosorbic acid, angelic acid, cinnamic acid, p-chlorocinnamic acid, $\beta$-stearylacrylic acid, itaconic acid, citroconic acid, mesaconic acid, glutaconic acid, aconitic acid, maleic acid, fumaric acid, tricarboxyethylene, and maleic anhydride.

Preferred superabsorbent polymers contain carboxyl groups, such as the above-described carboxylic acid/ carboxylate containing groups, or salts thereof. These polymers include hydrolyzed starch-acrylonitrile graft copolymers, partially neutralized hydrolyzed starch-acrylonitrile graft copolymers, starch-acrylic acid graft copolymers, partially neutralized starch-acrylic acid graft copolymers, hydrolyzed vinyl acetate-acrylic ester copolymers, hydrolyzed acrylonitrile or acrylamide copolymers, slightly network crosslinked polymers of any of the aforementioned copolymers, or highly preferred are polyacrylic acid, and/or salts thereof, and slightly network crosslinked polymers of polyacrylic acid/ salts. These polymers can be used either solely or in the form of a mixture of two or more different polymers. Examples of these polymer materials are disclosed in U.S. Patent 3,661,875, U.S. Patent 4,076,663, U.S. Patent 4,093,776, U.S. Patent 4,666,983, and U.S. Patent 4,734,478.

Most preferred polymer materials used for making the superabsorbent polymers herein are polyacrylates/ acrylic acids and derivatives and salts thereof, preferably (slightly) network crosslinked polymers partially neutralized polyacrylic acids/ salts and/ or starch derivatives thereof. Preferred may be that partially neutralized polymeric acrylic acid is used in the process herein.

The superabsorbent polymers useful in the present invention can be formed by any polymerization and/or crosslinking techniques. Typical processes for producing these polymers are described in U.S. Reissue Patent 32,649 (Brandt et al.), issued April 19, 1988, U.S. Patent 4,666,983 (Tsubakimoto et al.), issued May 19, 1987, and U.S. Patent 4,625,001 (Tsubakimoto et al.), issued November 25, 1986; US 5,140,076 (Harada); US 6,376,618 B1, US 6,391,451 and US 6,239,230 (Mitchell); US 6,150,469 (Harada). Crosslinking can be affected during polymerization by incorporation of suitable crosslinking monomers. Alternatively, the polymers can be crosslinked after polymerization by reaction with a suitable reactive crosslinking agent. Surface crosslinking of the initially formed polymers is a preferred way to control to some extent the absorbent capacity, porosity and permeability.

The superabsorbent polymers may also be surface-crosslinked, prior to, simultaneously with or after the coating step of combining, mixing or coating the superabsorbent polymers with the material with radiation-induced or inducible hydrophilicity. Suitable general methods for carrying out surface crosslinking of absorbent polymers herein are disclosed in U.S. Patent 4,541,871 (Obayashi), issued September 17, 1985; published PCT application WO92/16565 (Stanley), published October 1, 1992, published PCT application WO90/08789 (Tai), published August 9, 1990; published PCT application WO93/05080 (Stanley), published March 18, 1993; U.S. Patent 4,824,901 (Alexander), issued April 25, 1989; U.S. Patent 4,789,861 (Johnson), issued January 17, 1989; U.S. Patent 4,587,308 (Makita), issued May 6, 1986; U.S. Patent 4,734,478 (Tsubakimoto), issued March 29, 1988; U.S. Patent 5,164,459 (Kimura et al.), issued November 17, 1992; published German patent application 4,020,780 (Dahmen), published August 29, 1991; ; US 5,140,076 (Harada); US 6,376,618 B1, US 6,391,451 and US 6,239,230 (Mitchell); US 6,150,469 (Harada); and published European patent application 509,708 (Gartner), published October 21, 1992.

Most preferably, the superabsorbent polymers comprise from about 50% to 95% (mol percentage) neutralized, preferably about 75% neutralized, (slightly cross-linked) polyacrylic acid (e.g.. poly (sodium acrylate/acrylic acid)).

The superabsorbent polymers preferably have a low amount of extractables, preferably less than 15% (by weight of the polymers; 1 hour test value), more preferably less than 10% and more preferably less than 5%, or even more preferably less than 3% by weight. The extractables and levels thereof and determination thereof is further described in for example US5,599,335; US5,562,646 or US5,669,894.

Surface modifying agent

In one embodiment of the invention the superabsorbent material comprises superabsorbent polymers combined, or coated with a surface modifying material. Said surface modifying material is such that it provides the required ratio in Fluid Rise Height (FRH) of at least 1.10, as described herein. It has surprisingly been found that when the superabsorbent material has a ratio in Fluid Rise Height ratio of at least 1.10, as defmed herein, the superabsorbent material can provide improved liquid transport, whilst still providing excellent absorbency, swelling and permeability, e.g. high SFC *and* CRC values.

It typically modifies the surface of the superabsorbent polymers such that the surface becomes highly hydrophilic. The surface modifying material is typically (highly) hydrophilic. It preferably forms a particulate or film coating on the surface of the superabsorbent polymers.

The surface modifying material may be a particulate material, for example a crystalline material, and/or an inorganic material and/ or a non-polymeric organic material.

In one embodiment, the surface-modifying agent is a material with radiation-induced hydrophilicity.

Material with radiation-induced hydrophilicity

The superabsorbent material herein comprises a material with radiation-induced hydrophilicity, formed by combining the superabsorbent polymers with said material or by combining the superabsorbent polymers with a material with radiation inducible hydrophilicity, and then activating the material with radiation.

Said material is such that it has an increased hydrophilicity after radiation with suitable radiation, compared to prior to activation by radiation.

Said material with radiation-induced hydrophilicity, when present in the absorbent article is preferably a solid material, in the form of particles. Said particles preferably form a partial or complete and/ or homogeneous coating on the surface of the superabsorbent polymer particles.

Radiation-induced hydrophilicity is a phenomenon known in the art, as for example described in American Chemical Society, Chem. Mater. 2002, 14, 2812-2816. Without being bound by theory, it is believed that radiation-inducible hydrophilicity is based on structural changes of the surface of said material with radiation induced hydrophilicity. It is thought that by radiation at energies similar or higher than the band gap of said material, (e.g. UV light, Ar+ ion beam) oxygen vacancies on the material surface are generated, e.g. after electron transfer from the valence band to the conduction band, which then coordinate to water or hydroxyl groups. This is believed to render the induced material more hydrophilic.

In addition, and with respect to the present invention, it is also believed that in case said material also shows radiation-(or photo-)catalytic oxidation, such as $TiO_2$, $SnO_2$, ZnO, hydroxyl radicals (OH. ) might be formed. The hydroxyl radicals might oxidize contaminating components and/or oxidize organic structures of the superabsorbent polymers. Both might further increase the hydrophilicity and wettability of the superabsorbent polymers and superabsorbent material. ,

The materials with radiation-induced or inducible hydrophilicity are semiconductors, preferably inorganic semiconductors. The radiation suitable to activate said material herein may be UV light, UV/visible light, Ar+ ion beams, electron-beam irradiation or so-called low density electron beams (such as between 0.48 keV and 1.0 keV electron beams).

Preferred are materials that are activated or induced by UV/ visible radiation, typically of a wave length of from 200 nm to 480 nm, or more preferably from 300 nm to 480 nm, or more preferably from 350 nm to 480 nm.

Preferred materials herein include $TiO_2$, $SnO_2$, ZnO, $WO_3$, $V_2O_5$, or mixture thereof, and highly preferred may be to include at least, or only, $TiO_2$.

It may be advantageous to include a material with radiation-induced hydrophilicity that also provides further benefits, e.g. color enhancement (e.g. $TiO_2$), or pharmaceutical activity (e.g. ZnO).

In some embodiments herein, the material with radiation-induced or -inducible hydrophilicity forms a coating on the superabsorbent polymer.

The term coating, as used herein, includes coatings that completely cover a surface (e.g., continuous coatings, including those that form films on the surface), as well as coatings that may only partially cover a surface (e.g., discontinuous coatings, for example coatings that have a gap, for example due to the drying step of the coating process). The latter category of coatings may include coatings obtained by a distribution of particles of the coating material on the surface of a superabsorbent polymer particle with spaces between said particles of the coating material. In some embodiments, the coating is preferably in the form of at least one layer of particles of said coating material on the surface of the superabsorbent polymer particle, and is a substantially uniform layer and/ or a uniform distribution of particles of said coating material on said superabsorbent polymer particle surface.

Said material with radiation-induced or -inducible hydrophilicity comprises preferably fine particles, e.g. primary, discrete particles with a largest particle size (dimension, e.g. diameter) of less than 10 $\mu$m, preferably less than 5 $\mu$m, or even more preferably less than 0.5 $\mu$m (as may be determined by known methods, such as ESEM ; Environmental Scanning Electron Microscopy).

However, the material may comprise agglomerates of such primary, discrete particles, with a larger dimension.

Said material may also be formed in situ from a prescursor, for example by hydrolysis of a precursor by thermolysis of a precursor, or by chemical vapour deposition of a precursor.

Simultaneously with or subsequently to the in-situ formation, the resulting material with radiation-inducible hydrophilicity may be induced by exposure to radiation.

For example, $TiO_2$ can be formed via hydrolysis of $TiCl_4$ or $Ti(OR)_4$ (whereby R is a suitable organic group, such as iso-propyl); $SnO_2$ can be formed via hydrolysis of $TiCl_4$, $SnCl_4$, or $Sn(OR)_4$ or $Ti(OR)_4$ (whereby R is a suitable organic group, such as iso-propyl); $V_2O_5$ can be formed by hydrolysis of $VOCl_3$.

Process of activation and formation of the superabsorbent material

The material with radiation-inducible hydrophilicity is submitted herein to suitable radiation, as described above, for example UV/ visible light, to activate the material and render it more hydrophilic. It is combined, for example intimately mixed or even coated on the superabsorbent polymers herein, to render the resulting superabsorbent material more hydrophilic.

In one embodiment, the radiation is applied such that its energy is transferred homogeneous to said material.

In one embodiment, thin layers or even monolayers of the combination of the material with radiation-inducible hydrophilicity and the superabsorbent polymer are exposed to the appropriate radiation to activate said material with radiation-inducible hydrophilicity.

In another embodiment, the combination of the superabsorbent polymers and the material with radiation-inducible hydrophilicity is agitated during the exposure to the radiation, appropriate to activate said material with radiation-inducible hydrophilicity.

In yet another embodiment, the combination of the superabsorbent polymers and the material with radiation-inducible hydrophilicity is moving in a way that a thin moving bed of thereof is formed (e.g. with thickness of less than 20 particle diameters, more preferably less than 5 particle diameters) during the exposure to said radiation, appropriate to activate said material with radiation-inducible hydrophilicity.

However, the radiation step may be done prior, simultaneous with or subsequent to combining of said (inducible or induced) material with said superabsorbent polymers, and/or prior to, simultaneous with or subsequently to incorporating the superabsorbent material into the articles herein. In one embodiment, the radiation activation step is performed on the material with radiation-inducible hydrophilicity prior to combining this with the superabsorbent polymers. In one embodiment, the superabsorbent material with superabsorbent polymers and a material with radiation-inducible hydrophilicity are incorporated in part of an absorbent article, and subsequently radiated or partially radiated, to create zones with radiation induced hydrophilcity and zones without radiation-induced hydrophilicity. This may be useful to help to direct liquid absorption and/ or distribution.

The superabsorbent polymers and said material with radiation-induced or -inducible hydrophilicity may be combined by any method. This includes mixing, such as dry blending, and coating processes, such as dispersion coating. Preferred may be that a dispersion of said material in a liquid, for example water, is applied to the polymers, for example by use of a fluidized bed, where after the liquid may be removed, for example by drying, so deliver a fine particle coating of said material on the surface of the superabsorbent polymer particles, as described herein above.

In one embodiment, the superabsorbent polymers may be surface cross-linked, as described herein above, prior to the radiation-activation step herein.

The absorbent article may be formed by any known process in the art.

In one embodiment herein, the absorbent/ storage core or layer of the article comprises the superabsorbent material herein, preferably combined with a matrix material, such as cellulose fibers, or more preferably such as thermoplastic/ adhesive fibers, as described above. This may be formed by mixing the ingredients and placing this between one or more core covers, or core wrapping material, or by forming a laminate of these ingredients between one or more core covers. Then a laminate of said superabsorbent material, matrix fibers between one or more core covers is obtained.

Examples

The following superabsorbent polymer samples were prepared and subsequently mixed with a material with radiation-inducible hydrophilicity and subsequently induced to result in the superabsorbent material useful for the absorbent articles herein.

Superabsorbent base polymer (for preparation of sample 868)

A 16000 ml resin kettle (equipped with a four-necked glass cover closed with septa, suited for the introduction of a thermometer, syringe needles) is charged with about 5 kg ice (4845.02 g) (prepared from de-ionized water). Typically, a magnetic stirrer, capable of mixing the whole content (when liquid), is added. 1110.18 g 50% NaOH solution (for analysis, from Merck) is added to the ice and the resulting slurry is stirred. An amount of glacial acrylic acid (AA) (appr. 340 g) is taken from 2000.00 g AA (for synthesis, from Merck) to dissolve 42.79 g MethyleneBisAcrylAmide (MBAA) (from Sigma Aldrich). The remaining AA is added to the ice/NaOH mixture while stirring is continued. A thermometer is introduced and once the temperature starts to drop (after neutralization) ice (prepared from de-ionized water) is added till the temperature is in the range of 15-25°C. 2000 mg initiator "V50" (= 2,2'-azobis (N,N'-dimethyleneisobutyramidine) dihydrochloride, from Waco Chemicals) are dissolved in appr. 35 g de-ionized water.

De-ionized water (the required amount to achieve in total 6845.02 g (ice + water) minus an amount (typically 1000 ml) to wash out the beakers that contain the MBAA solution and the initiator solution) is added. The MBAA solution and the initiator "V50" solution are added. Residual solution (MBAA solution and/or initiator "V50" solution) in the beakers is washed with the remaining water into the resin kettle.

Then, the resin kettle is closed, and a pressure relief is provided e.g. by puncturing two syringe needles through the septa. The solution is then purged vigorously with argon via a 60 cm injection needle while stirring at about 600 - 1200 RPM. The argon stream is placed close to the stirrer for efficient and fast removal of dissolved oxygen. Stirring is discontinued after about 10 minutes, while argon purging is continued, and after about another 5 minutes two photo lamps (e.g. TWINLITE from Balcar, equipped with PL-L830/4P, 36 W lamps from Phillips) are placed on either side of the vessel. The solution typically starts to become turbid after 2-5 minutes. Then, the argon injection needle is raised above the surface of the gel and purging with argon is continued at a reduced flow rate. Once the temperature rises the resin kettle might be turned in a 90° turn around the vertical axis, so each quarter of the resin kettle is exposed to the light and the temperature rises similarly in the different parts of the resin kettle. Turning might be repeated every 10 minutes to ensure similar temperature evolution in each quarter of the resin kettle. The temperature is monitored; typically it rises from 20°C to 60 - 70°C within 60 - 90 minutes. Once the temperature reaches about 60°C, the lamps are switched off. Once the temperature starts to drop, the resin kettle is transferred into a circulation oven and kept at 60°C for 15 - 18 hours.

After this time, the resin kettle is allowed to cool, and the gel is removed into a flat glass dish. The gel is then broken or cut with scissors into smaller pieces. The gel is grinded with a grinder (e.g. X 70 G from Scharfen), put into a flat glass dish and transferred into a vacuum oven (e.g. Vacutherm, VT6130 P-BL, Heraeus, equipped with vapour trap e.g. Titan Vapor Trap, Kinetics, and/or equipped with vacuum pump e.g. Trivac®, Leybold), where it is dried. Typically, the material is dried as following: 1-2 days at 50°C/ max. vacuum, then 3-4 hours at 60°C/ max. vacuum, then 3-4 hours at 70°C/ max. vacuum, then 3-18 hours at 85°C/ max. vacuum and finally 15-18 h at 100°C/ max. vacuum till a pressure of about 1-3 mbar is achieved.

Once the gel has reached a constant weight (usually 3 days drying procedure), it is ground using a mechanical mill (e.g. IKA mill MF 10.1), and sieved to 150 - 600 $\mu$m (e.g. with AS 400 control from Retsch).

Details of the milling procedure:

The dried superabsorbent base polymer of above is milled using a mechanical mill (IKA mill MF 10.1) with an interchangeable sieve with 3 mm opening and sieved to 150 - 600 $\mu$m (e.g. with AS 400 control from Retsch). The fraction > 600 $\mu$m is again milled with an interchangeable sieve with 3 mm opening and sieved to 150 - 600 $\mu$m. After that, the remaining fraction > 600 $\mu$m is milled with an interchangeable sieve with 2 mm opening and sieved to 150 - 600 $\mu$m. The remaining fraction > 600 $\mu$m is again milled with an interchangeable sieve with 2 mm opening and sieved to 150 - 600 $\mu$m. After that, the remaining fraction > 600 $\mu$m is milled with an interchangeable sieve with 1.5 mm opening and sieved to 150 - 600 $\mu$m. The remaining fraction > 600 $\mu$m is again milled with an interchangeable sieve with 1.5 mm opening and sieved to 150 - 600 $\mu$m. All fractions 150-600 $\mu$m are collected and combined to form the base polymer sample.

The residual moisture of the sample is then determined. In case this is more than 6% by weight, the sample is again dried at 100C/ max. vacuum, as above.

Surface Crosslinking to obtain sample 868

100 g portions of the dry, milled superabsorbent base polymer (as described above) are surface cross-linked as follows.

A 250 ml plastic beaker is equipped with a mechanical stirrer with a plastic or teflon blade, and charged with 100 g of a dry superabsorbent base polymer (in particulate form). The mechanical stirrer is selected in such a way that a good fluidization of the polymers can be obtained at 300 - 500 RPM. A 3000 microlitre syringe is charged with 3000 microlitre of a 4% solution (w/w) of Denacol (= EthyleneGlycolDiGlycidylEther = EGDGE) in 1,2-propanediol (prepared from 1.6 g 50% EGDGE solution (Sigma Aldrich) and 20 g 1,2-propanediol); another 10 ml syringe is charged with 7.5 ml de-ionized water.

The superabsorbent polymers are fluidized in the beaker at 500 RPM, and the surface cross-linking agent is added dropwise within 30 seconds. Mixing is continued for a total of three minutes. While stirring is continued, 7.5 ml of water are then added within 3 - 5 seconds, and stirring is continued at 300 - 500 RPM for another 3 minutes. After this time, the mixture is transferred into a glass beaker, sealed with aluminum foil, and is equilibrated for 0.5 hour. Then the beaker is transferred to a 140°C oven, and kept at this temperature for 120 minutes. After this time, the beaker is allowed to cool down, the contents are removed, and the surface cross-linked superabsorbent polymer is obtained. Any agglomerates may be carefully broken by gentle mechanical action. The resulting surface cross-linked superabsorbent polymer particles are then sieved manually to 150-600 micrometers. The resulting material of several surface cross-linking steps can be combined to obtain larger samples.

The obtained surface cross-linked superabsorbent polymer particles are dried at 100°C/ max. vacuum for 15-18 hours (resulting in sample 868).

Sample 868 had a SFC of 314 units ($cm^3 s 10^{-7}/g$) and a CRC of 18.9 g/g.

Superabsorbent Base Polymers for samples 865 and 866:

A 16000 ml resin kettle (equipped with a four-necked glass cover closed with septa, suited for the introduction of a thermometer, syringe needles) is charged with about 5 kg ice (5799.09 g) (prepared from de-ionized water). Typically, a magnetic stirrer, capable of mixing the whole content (when liquid), is added. 2207.97 g 50% NaOH solution (for analysis, from Merck) is added to the ice and the resulting slurry is stirred. An amount of glacial acrylic acid (AA) (appr. 450 g) is taken from 2651.76 g AA (for synthesis, from Merck) to dissolve 56.74 g MethyleneBisAcrylAmide (MBAA) (from Sigma Aldrich). The remaining AA is added to the ice/NaOH mixture while stirring is continued. A thermometer is introduced and once the temperature starts to drop (after neutralization) ice (prepared from de-ionized water) is added till the temperature is in the range of 15-25°C. 2652 mg initiator "V50" (= 2,2'-azobis (N,N'-dimethyleneisobutyramidine) dihydrochloride, from Waco Chemicals) are dissolved in appr. 35 g de-ionized water.

De-ionized water (the required amount to achieve in total 8339.69 g (ice + water) minus an amount (typically 1000 ml) to wash out the beakers that contain the MBAA solution and the initiator solution) is added. The MBAA solution and the initiator "V50" solution are added. Residual solution (MBAA solution and/or initiator "V50" solution) in the beakers is washed with the remaining water into the resin kettle.

Then, the resin kettle is closed, and a pressure relief is provided e.g. by puncturing two syringe needles through the septa. The solution is then purged vigorously with argon via a 60 cm injection needle while stirring at about 600 - 1200 RPM. The argon stream is placed close to the stirrer for efficient and fast removal of dissolved oxygen. Stirring is discontinued after about 10 minutes, while argon purging is continued, and after additional about 5 minutes two photo lamps (e.g. TWINLITE from Balcar, equipped with PL-L830/4P, 36 W lamps from Phillips) are placed on either side of the vessel. The solution typically starts to become turbid after 2-5 minutes. Then, the argon injection needle is raised above the surface of the gel and purging with argon is continued at a reduced flow rate. Once the temperature rises to about 35°C the resin kettle is turned in a 90° turn around the vertical axis, so each quarter of the resin kettle is exposed to the light and the temperature rises similarly in the different parts of the resin kettle. Turning might be repeated every 10 minutes to ensure similar temperature evolution in each quarter of the resin kettle. The temperature is monitored; typically it rises from 20°C to 60 - 70°C within 60 - 90 minutes. Once the temperature reaches about 60°C, the lamps are switched off. Once the temperature starts to drop, the resin kettle is transferred into a circulation oven and kept at 60°C for 15 -18 hours.

After this time, the resin kettle is allowed to cool, and the gel is removed into a flat glass dish. The gel is then broken or cut with scissors into smaller pieces. The gel is grinded with a grinder (e.g. X 70 G from Scharfen), put into a flat glass dish and transferred into a vacuum oven (e.g. Vacutherm, VT6130 P-BL, Heraeus, equipped with vapour trap e.g. Titan Vapor Trap, Kinetics, and/or equipped with vacuum pump e.g. Trivac®, Leybold), where it is dried. Typically, the material is dried as following: 1-2 days at 50°C/ max. vacuum, then 3-4 hours at 60°C/ max. vacuum, then 3-4 hours at 70°C/ max. vacuum, then 3-18 hours at 85°C/ max. vacuum and finally 15-18 h at 100°C/ max. vacuum till a pressure of - 1-3 mbar is achieved.

Once the gel has reached a constant weight (usually 3 days), it is ground using a mechanical mill (e.g. IKA mill MF 10.1), and sieved to 150 - 850 $\mu$m (e.g. with AS 400 control from Retsch).

Details of the milling procedure:

The dried superabsorbent base polymer (as obtained above) is milled using a mechanical mill (IKA mill MF 10.1) with an interchangeable sieve with 3 mm opening and sieved to 150 - 850 $\mu$m (e.g. with AS 400 control from Retsch). The fraction > 850 $\mu$m is again milled with an interchangeable sieve with 3 mm opening and sieved to 150 - 850 $\mu$m. After that, the remaining fraction > 850 $\mu$m is milled with an interchangeable sieve with 2 mm opening and sieved to 150 - 850 $\mu$m. The remaining fraction > 850 $\mu$m is again milled with an interchangeable sieve with 2 mm opening and sieved to 150 - 850 $\mu$m. After that, the remaining fraction > 850 $\mu$m is milled with an interchangeable sieve with 1.5 mm opening and sieved to 150 - 850 $\mu$m. The remaining fraction > 850 $\mu$m is again milled with an interchangeable sieve with 1.5 mm opening and sieved to 150 - 850 $\mu$m. All fractions 150-850 $\mu$m are collected and combine to form the superabsorbent base polymer sample.

The residual moisture of the sample is then determined. In case this is more than 6% by weight, the sample is again dried at 100C/ max. vacuum, as above.

Surface Crosslinking to obtain sample 865

100 g portions of the dry, milled superabsorbent base polymer (as described above) are surface cross-linked as follows.

A 250 ml plastic beaker is equipped with a mechanical stirrer with a plastic or teflon blade, and charged with 100 g of a dry superabsorbent base polymer (in particulate form). The mechanical stirrer is selected in such a way that a good fluidization of the polymers can be obtained at 300 - 500 RPM. A 1000 microlitre syringe is charged with 1000 microlitre of a 4% solution (w/w) of Denacol (= EthyleneGlycolDiGlycidylEther = EGDGE) in 1,2-propanediol (prepared from 1.6 g 50% EGDGE solution (Sigma Aldrich) and 20 g 1,2-propanediol); another 10 ml syringe is charged with 7.5 ml de-ionized water.

The superabsorbent polymers are fluidized in the beaker at 500 RPM, and the surface cross-linking agent is added dropwise within 30 seconds. Mixing is continued for a total of three minutes. While stirring is continued, 7.5 ml of water are then added within 3 - 5 seconds, and stirring is continued at 300 - 500 RPM for another 3 minutes. After this time, the mixture is transferred into a glass beaker, sealed with aluminum foil, and is equilibrated for 0.5 hour. Then the beaker is transferred to a 140°C oven, and kept at this temperature for 120 minutes. After this time, the beaker is allowed to cool down, the contents are removed, and the surface cross-linked superabsorbent polymer is obtained. Any agglomerates may be carefully broken by gentle mechanical action. The resulting surface cross-linked superabsorbent polymer particles are then sieved manually to 150-850 micrometer. The resulting material of several surface cross-linking steps can be combined to obtain larger samples.

The obtained surface cross-linked superabsorbent polymers are then dried at 100°C/ max. vacuum for 15-18 hours (e.g. Vacutherm, VT6130 P-BL, Heraeus equipped with vapour trap e.g. Titan Vapor Trap, Kinetics, and/or equipped with vacuum pump e.g. Trivac®, Leybold), to obtain sample 865.

Sample 865 had a SFC of 99 units ($cm^3s10^{-7}$/g) and a CRC of 22.3 g/g.

Surface Crosslinking to obtain sample 866

100 g portions of the same type dry, milled superabsorbent base polymer (as described above) are surface cross-linked as follows.

A 250 ml plastic beaker is equipped with a mechanical stirrer with a plastic or teflon blade, and charged with 100 g of a dry superabsorbent polymer (e.g. base polymer) in particulate form. The mechanical stirrer is selected in such a way that a good fluidization of the polymers can be obtained at 300 - 500 RPM. A 3000 microlitre syringe is charged with 1500 microlitre of a 4% solution (w/w) of Denacol (= EthyleneGlycolDiGlycidylEther = EGDGE) in 1,2-propanediol (prepared from 1.6 g 50% EGDGE solution (Sigma Aldrich) and 20 g 1,2-propanediol); another 10 ml syringe is charged with 7.5 ml de-ionized water.

The superabsorbent polymers are fluidized in the beaker at 500 RPM, and the surface cross-linking agent is added dropwise within 30 seconds. Mixing is continued for a total of three minutes. While stirring is continued, 7.5 ml of water are then added within 3 - 5 seconds, and stirring is continued at 300 - 500 RPM for another 3 minutes. After this time, the mixture is transferred into a glass beaker, sealed with aluminum foil, and is equilibrated for 0.5 hour. Then the beaker is transferred to a 140°C oven, and kept at this temperature for 120 minutes. After this time, the beaker is allowed to cool down, the contents are removed, and the surface cross-linked superabsorbent polymer is obtained. Any agglomerates may be carefully broken by gentle mechanical action. The resulting surface cross-linked superabsorbent polymer particles are then sieved manually to 150-850 micrometer.

The obtained surface cross-linked superabsorbent polymer are combined and dried at 100°C/ max. vacuum for 15-18 hours (e.g. Vacutherm, VT6130 P-BL, Heraeus equipped with vapour trap e.g. Titan Vapor Trap, Kinetics, and/or equipped with vacuum pump e.g. Trivac®, Leybold), to obtain the sample 866.

The residual moisture of the sample is then determined. In case this is more than 6% by weight, the sample is again dried at 100C/ max. vacuum, as above.

Sample 866 had a SFC of 199 units ($cm^3s10^{-7}$/g) and a CRC of 20.4 g/g.

Coating of sample 868 with $TiO_2$ to obtain sample 4563

118.84 g superabsorbent polymer sample 868 is put into a 250 ml PE bottle (VWR International, Bottle wide neck + cap round, LDPE, 250 ml) and 1.21 g $TiO_2$ powder (Sigma Aldrich, Titanium(IV)oxide, powder, < 5 micrometer, 99.9+%) is added. The bottle is closed and tumbled for 2.5 hours in a tumbling mixer (Turbula®, System Schatz, Willy A. Bachofen AG, Maschinenfabrik, Basel, Switzerland).

40 g of the mixture are taken and divided on a rotary sample divider (Rotary Micro Riffler, Quantachrome Instruments) into 8 portions. The sample dividing is repeated with a second sample of 40g, so 16 chemical tubes containing approximately 5 g of the mixture are obtained. 4 tubes of each sample dividing procedure are taken, combined and stored in a clean PE bottle for reference.

Sample 4563 had a SFC of 387 units ($cm^3s10^{-7}$/g) and a CRC of 19.3 g/g.

Radiation (irradiation) of sample 4563 to obtain sample 4582 (superabsorbent material as used in the invention)

The mixture in the remaining 8 tubes (sample 4563 material) is exposed to radiation (irradiation). The content of one of the tubes (appr. 5 g) is distributed evenly in a glass Petri dish (inner diameter appr. 120 mm) and exposed to UV light of appr. 300-420 nm for 30 minutes. For radiation e.g. the light source "Polimer Apparatus, GR.E.500W", with lamp GN 500 Zs and equipped with a ventilation chamber (Helios Italquartz) can be used. The typical distance between the light source and the sample is about 20 to 35 cm. After irradiation, the irradiated, radiation-induced material is stored in stoppered chemical tubes. The irradiation procedure is repeated untill the content of all remaining tubes has been exposed to irradiation. The irradiated, radiation-induced superabsorbent material is combined in a PE bottle (VWR International, Bottle wide neck + cap round, LDPE, 250 ml) and mixed by turning the PE bottle upside down several times (5-10 times) (to obtain sample 4582).

Sample 4582 had a SFC of 356 units ($cm^3s10^{-7}$/g) and a CRC of 19.0 g/g.

Coating of sample 865 with $TiO_2$ to obtain sample 4583

118.47 g superabsorbent polymer sample 865 is put into a 250 ml PE bottle (VWR International, Bottle wide neck + cap round, LDPE, 250 ml) and 1.21 g $TiO_2$ powder (Sigma Aldrich, Titanium(IV)oxide, powder, < 5 micrometer, 99.9+%) is added. The bottle is closed and tumbled for 2.5 hours in a tumbling mixer (Turbula®, System Schatz, Willy A. Bachofen AG, Maschinenfabrik, Basel, Switzerland).

40 g of the mixture are taken and divided on a rotary sample divider (Rotary Micro Riffler, Quantachrome Instruments) into 8 portions. The sample dividing is repeated with a second sample of 40 g, so 16 chemical tubes containing approximately 5 g of the mixture are obtained. 4 tubes of each sample dividing procedure are taken, combined and stored in a clean PE bottle for reference (sample 4583).

Sample 4583 had a SFC of 136 units ($cm^3s10^{-7}$/g) and a CRC of 22.7 g/g.

Radiation (rradiation) of sample 4583 to obtain sample 4584:

The mixture in the remaining 8 tubes (sample 4583) is exposed to radiation (irradiation). The content of one of the tubes (appr. 5 g) is distributed evenly in a glass Petri dish (inner diameter appr. 120 mm) and exposed to UV light of appr. 300-420 nm for 30 minutes. For irradiation, e.g. the light source "Polimer Apparatus, GR.E.500W", with lamp GN 500 Zs and equipped with a ventilation chamber (Helios Italquartz) can be used. The typical distance between the light source and the sample is about 20 to 35 cm. After irradiation, the irradiated, radiation-induced material is stored in stoppered chemical tubes. The irradiation procedure is repeated until the content of all remaining tubes has been exposed to irradiation. The irradiated, radiation-induced superabsorbent material is combined in a PE bottle (VWR International, Bottle wide neck + cap round, LDPE, 250 ml) and mixed by turning the PE bottle upside down several times (5-10 times), to obtain sample 4584.

Sample 4584 had a SFC of 146 units ($cm^3s10^{-7}$/g) and a CRC of 22.7 g/g.

Coating of sample 866 with $TiO_2$ to obtain sample 4585

118.35 g superabsorbent polymer sample 866 is put into a 250 ml PE bottle (VWR International, Bottle wide neck + cap round, LDPE, 250 ml) and 1.11 g $TiO_2$ powder (Sigma Aldrich, Titanium(IV)oxide, powder, < 5 micrometer, 99.9+%) is added. The bottle is closed and tumbled for 2.5 hours in a tumbling mixer (Turbula®, System Schatz, Willy A. Bachofen AG, Maschinenfabrik, Basel, Switzerland).

40 g of the mixture are taken and divided on a rotary sample divider (Rotary Micro Riffler, Quantachrome Instruments) into 8 portions. The sample dividing is repeated with a second sample of 40 g, so 16 chemical tubes, each containing approximately 5 g of the mixture are obtained. 4 tubes of each sample dividing procedure are taken, combined and stored in a clean PE bottle for reference, to obtain sample 4585.

Sample 458 had a SFC of 246 units ($cm^3s10^{-7}$/g) and a CRC of 21.6 g/g.

Radiation (irradiation) of sample 4585 to obtain sample 4589

The mixture in the remaining 8 tubes (sample 4585) is exposed to radiation (irradiation). The content of one of the tubes (appr. 5 g) is distributed evenly in a glass Petri dish (inner diameter appr. 120 mm) and exposed to UV light of appr. 300-420 nm for 30 minutes. For irradiation e.g. the light source "Polimer Apparatus, GR.E.500W", with lamp GN 500 Zs and equipped with a ventilation chamber (Helios Italquartz) can be used. The typical distance between light source and irradiated sample is about 20-35 cm. After irradiation, the irradiated, radiation-induced superabsorbent material is stored in stoppered chemical tubes. The irradiation procedure is repeated until the content of all remaining tubes has been exposed to irradiation. The irradiated, radiation-induced superabsorbent material is combined in a PE bottle (VWR International, Bottle wide neck + cap round, LDPE, 250 ml) and mixed by turning the PE bottle upside down several times (5-10 times) (sample 4589).
Sample 4589 had a SFC of 276 units ($cm^3s10^{-7}/g$) and a CRC of 21.5 g/g.

Coating of sample 865 with $TiO_2$ to obtain sample 4586

118.15 g superabsorbent polymer sample 865 is put into a 250 ml PE bottle (VWR International, Bottle wide neck + cap round, LDPE, 250 ml) and 1.11 g $TiO_2$ powder (Sigma Aldrich, Titanium(IV)oxide, powder, < 5 micrometer, 99.9+%) is added. The bottle is closed and tumbled for 2.5 hours in a tumbling mixer (Turbula®, System Schatz, Willy A. Bachofen AG, Maschinenfabrik, Basel, Switzerland). 40 g of the mixture are taken and divided on a rotary sample divider (Rotary Micro Riffler, Quantachrome Instruments) into 8 portions. The sample dividing is repeated with a second sample of 40 g, so 16 chemical tubes, each containing approximately 5 g of the mixture are obtained. 4 tubes of each sample dividing procedure are taken, combined and stored in a clean PE bottle for reference (sample 4586).
Sample 4586 had a SFC of 152 units ($cm^3s10^{-7}/g$) and a CRC of 22.6 g/g.

Radiation (irradiation) of sample 4586 to obtain sample 4590

The mixture in the remaining 8 tubes (sample 4586) is exposed to radiation (irradiation). The content of one of the tubes (appr. 5 g) is distributed evenly in a glass Petri dish (inner diameter appr. 120 mm) and exposed to UV light of appr. 300-420 nm for 30 minutes. For irradiation e.g. the light source "Polimer Apparatus, GR.E.500W", with lamp GN 500 Zs and equipped with a ventilation chamber (Helios Italquartz) can be used. The typical distance between light source and irradiated sample is about 20-35 cm. After irradiation, the irradiated, radiation-induced superabsorbent material is stored in stoppered chemical tubes. The irradiation procedure is repeated until the content of all remaining tubes has been exposed to irradiation. The irradiated, radiation-induced superabsorbent material is combined in a PE bottle (VWR International, Bottle wide neck + cap round, LDPE, 250 ml) and mixed by turning the PE bottle upside down several times (5-10 times) (sample 4590).
Sample 4590 had a SFC of 156 units ($cm^3s10^{-7}/g$) and a CRC of 22.7 g/g.
N.B.: The samples 4563, 4582, 4583, 4584, 4585, 4586, 4589 and 4590 all had a moisture-content in the range of 0.25% to 0.75%.

RHH and FRH ratio test results of the samples

| Parameter | 868 | 4563 | 4582 |
|---|---|---|---|
| Rise in height n-heptane (RHH) [cm] | 7.2 | 7.9 | 7.7 |
| Rise in height 25% saline [cm] | 4.3 | 5.3 | 11.5 |
| FRH ratio | 0.60 | 0.67 | 1.49 |

| Parameter | 865 | 4583 | 4584 |
|---|---|---|---|
| Rise in height n-heptane (RHH) [cm] | 5.4 | 5.7 | 5.7 |
| Rise in height 25% saline [cm] | 4.3 | 5.0 | 7.2 |
| FRH ratio | 0.80 | 0.88 | 1.26 |

| Parameter | 866 | 4585 | 4589 |
|---|---|---|---|
| Rise in height n-heptane (RHH) [cm] | 4.7 | 4.8 | 5.5 |
| Rise in height 25% saline [cm] | 4.0 | 4.5 | 6.7 |
| FRH ratio | 0.85 | 0.94 | 1.22 |

| Parameter | 865 | 4586 | 4590 |
|---|---|---|---|
| Rise in height n-heptane (RHH) [cm] | 5.4 | 5.4 | 5.1 |
| Rise in height 25% saline [cm] | 4.3 | 4.8 | 7.2 |
| FRH ratio | 0.80 | 0.89 | 1.41 |

TEST METODS

All tests are performed in a climate controlled room at standard conditions of 23°C (+/- 2°C) temperature and 50% relative humidity (+/- 5%).

Fluid rise height ratio (FRH ratio) and Rise in Height for n-Heptane (RHH)

The following method serves to determine the Fluid rise height ratio (FRH ratio) as used herein and the Rise in Height for n-Heptane (RHH), as used herein.

The FRH ratio is the ratio of the rise in height through a superabsorbent material sample of a 25% saline-solution rise height compared to the rise in height through a superabsorbent material sample of n-heptane.

The 25% saline solution is prepared such that the 25 % saline solution is free of contaminations that may impact the surface tension. The resulting pure 25% saline solution should have a surface tension of at least 78 mN/m, and typically about 82 mN/m. This can be measured with a KRUESS K10 ST tensiometer via the Wilhelmy plate method, as described in the user's manual (the beaker used should be completely clean, which can be tested by measuring the beaker with deionized water, which should have a surface tension of about 70-71 mN/m; the same beaker should then be washed with the 25% saline solution prior to measuring the surface tension of the saline solution).

For each test/ test solution a fresh sample of the superabsorbent material of the invention is used and clean equipment is used.

For sample division, a sample divider, e.g. a rotary sample divider like Rotary Micro Riffler, Quantachrome Instruments, is used to obtain representative samples of the desired amount, e.g. 5 g.

For each test, 5.0 g of the dry superabsorbent material (e.g. having the particle size distribution as the corresponding bulk material form, typically a particle size distribution as described herein) comprising less than 1% by weight of moisture (or an adjusted amount, see below)- herein referred to as "sample"- is filled completely into an acrylic-glass tube (inner diameter 8 mm, outer diameter 11 mm) of 200 mm length (or adjusted length- see below) resulting in a tube with superabsorbent material filled up to a certain fill height of which the bottom is covered with an acrylic-glass tube-cap (inner diameter 11 mm) of 15 mm length with stainless steel mesh bottom (as used for SFC or AAP equipment). The inner tube is marked at a height of 5 mm.

Adjustment of sample amount: in case the superabsorbent is such that the rise in height of n-heptane or the rise in height of 25% saline is within 5 mm (or less) from the fill height of the superabsorbent material (after tapping), the sample amount is multiplied by a factor of 2 (so the fill height of the superabsorbent material in the cup is then about twice as high). This adjustment of the sample amount can be repeated if required, e.g. 2 adjustments result in a sample amount of 20 g, with a fill height of about 4 about the original fill height.

Adjustment of the length of the acrylic glass tube (inner diameter 8 mm, outer diameter 11 mm): in case the fill height of superabsorbent material before tapping would exceed the length of the acrylic glass tube (inner diameter 8 mm, outer diameter 11 mm), i.e. the sample could not be filled completely into the closed tube, the length of the tube is multiplied by a factor of 2. The adjustment of the tube length can be repeated subsequently if required, e.g. 2 adjustments result in a tube length of 800 mm.

The exact weight of the superabsorbent material sample is recorded. The tube is tapped manually on the lab desk for 1 min at a frequency of about 2 taps per second and amplitude of 2-3 cm. The absorbent fill-height after tapping is recorded. A Petri dish with at least 110 mm diameter, and at least 12 mm height is filled with n-heptane, with a liquid fill-height of more than 5 mm. Clamps and supporting equipment are used to fix the tube vertically in and above the Petri dish. The filled tube after tapping is dipped with the bottom (with mesh) into n-heptane, so the n-heptane surface is at the same level as the mark of 5 mm on the tube (so the tube will not be in direct contact with the bottom of the Petri dish, but in direct contact with the n-heptane). It needs to be ensured that the n-heptane level remains at this level during the measurement. The liquid is allowed to rise through the superabsorbent material for 10 min. Then, the distance between the 5mm mark and the medium top level of raised n-heptane is measured and recorded, which is the rise in height for n-heptane (RHH). (The medium top level of raised liquid is detected visually, as average of highest point of liquid - dry superabsorbent material transition and lowest point of liquid - dry superabsorbent material transition. To ease the visual detection, an additional light source, e.g. a table lamp can be used.)

The test is repeated one time with a fresh sample, so an average rise in for n-heptane (RHH) is obtained. Similarly, a test tube with superabsorbent material is prepared as above, and, after tapping, placed into a 25% saline solution in a fresh Petri dish, so the saline surface is at the same level as the 5 mm mark on the tube. The saline level has to remain at this level during the measurement. The saline is allowed to rise for 10 min.

Then, as above, the distance between the 5 mm mark and the medium top level of raised liquid is measured and recorded; this is the rise in height for the 25% saline solution (RHS).

The test is repeated one time with a fresh sample, so an average rise in height for 25% saline solution (RHS) is obtained.

(It should be noted that the superabsorbent material does not noticeably absorb the n-heptane or 25% saline solution (with a liquid uptake of 2 g/g and lower), so that the RHH and RSH are not impacted by sorption and swelling characteristics of the superabsorbent material.)

Then, the ratio of RSH to RHH (the average rise in height of the 25% saline solution to the average rise in height of the n-heptane is determined) and this is herein reported as "Fluid Rise Height Ratio" (FRH ratio).

### Moisture or water content

European Disposables and Nonwovens Association (EDANA) method 430.2-02 (2002) is used herein to determine the moisture or water content of the superabsorbent material or polymers as referred to herein.

### Basis Weight

European Disposables and Nonwovens Association (EDANA) standard method for Mass per Unit Area (40.3-90) is suitable to obtain the basis weight of a region (of 20 mm x 15 mm) with the superabsorbent material herein.

### Caliper

European Disposables and Nonwovens Association (EDANA) standard method for Thickness (No 30.5-99) is suitable to measure the thickness of a region (20 mm x 15 mm) with superabsorbent material of the absorbent articles herein. A suitable apparatus is described in paragraph 4.1 thereof. The specified pressure is 2.1 kPa.

### Density

The density of a region with superabsorbent material herein is obtained by dividing the basis weight determined using the Basis Weight method above by the caliper determined by the Caliper method above.

### Centrifuge Retention Capacity of Superabsorbent material in the region(s)

European Disposables and Nonwovens Association (EDANA) standard method 441.2-02 is suitable to obtain the CRC of the superabsorbent material herein.

### Centrifuge Retention Capacity (CRC) of layer or region comprising superabsorbent material of an absorbent article

The CRC of region with the superabsorbent material herein can be determined with a modified EDANA standard test method 441.2-02. Namely, this standard test is applied on a cut-out sample of a region or layer (so the standard sample of 0.200g of superabsorbent powder used in this test is now replaced by a cut-out sample of the region or layer). The sample size of 20mm x 15 mm is used. The sample can be obtained by separating the distinctive layer/ region of the absorbent article. The sample of the dimensions specified above can then be cut with a die- cutter. Each sample should then be handled and measured as specified in the EDANA method, e.g. stored in closed container and conditioned prior to measurement. A CRC value per region (sample) will be obtained. If appropriate, a multitude of samples per absorbent structure or layer may be cut out and measured and an average CRC per structure or layer may be obtained.

Basis Capacity of a region

The basis capacity of a region is calculated by multiplying the CRC of said region with the basis weight of said region, as may be determined per the methods above:

$$\text{Basis capacity} = \text{CRC of region} * \text{Basis Weight of region}$$

Basis capacity is given in kg of fluid absorbed per $m^2$ of layer. Since density of 0.9% saline solution is about 1 gram / $cm^3$ it is typically reported in liter of fluid per m2 of layer ($l/m^2$), as also used herein.
An average basis capacity for an absorbent structure or layer may also be obtained as set out above.

Absorption Efficiency of a region

The absorption efficiency of a region is calculated by multiplying the CRC of the region with the density of said region, as may be determined per the methods set out above:

$$\text{Absorption Effectiveness} = \text{CRC of region} * \text{density of the region}$$

$$(\text{reported in ml} / cm^3)$$

An average basis capacity for an absorbent structure or layer may also be obtained as set out above.

**Claims**

1. An absorbent article comprising a superabsorbent material, comprising superabsorbent polymers and a semiconductor material with radiation-induced hydrophilicity, obtainable by

   a) combining said superabsorbent polymers and said material with radiation-induced hydrophilicity; or
   b) combining said superabsorbent polymers and a material with radiation-inducible hydrophilicity, to obtain a mixture and submitting said mixture to radiation suitable to induce said hydrophilicity.

2. An absorbent article as in claim 1, whereby said superabsorbent material having a Fluid Rise Height Ratio (FRH ratio) of at least 1.10.

3. An absorbent article as in claim 2, whereby the FRH ratio is 1.25 or more, or preferably 1.40 or more.

4. An absorbent article as in any of claims 2 or 3, whereby the Rise in Height of n-heptane (RHH) is from 5 cm to 19 cm.

5. An absorbent article as in any of preceding claims, whereby said material with radiation-induced hydrophilicity is present at a level of 0.01% to 2% by weight of the mixture of superabsorbent material.

6. An absorbent article as in any preceding claim, whereby said superabsorbent material has a moisture-content less than 2% by weight, preferably less than 1% by weight.

7. An article as in claim 1, whereby said radiation is electromagnetic radiation of a wave length of from 200nm to 480 nm.

8. An article as claim 1, whereby said material with radiation-induced hydrophilicity comprises $TiO_2$, $SnO_2$, ZnO, $WO_3$, $V_2O_5$, or mixture thereof, preferably at least $TiO_2$.

9. An absorbent article as in claim 1 whereby the material with radiation-induced hydrophilicity forms a coating on the surface of the superabsorbent polymers.

10. An absorbent article as in any preceding claim, comprising an absorbent structure with one ore more regions comprising said superabsorbent material and having a basis capacity of more than 6.5 l/m$^2$ and/ or an absorption efficiency of at least 3 ml/cm$^3$.

11. An absorbent article as in any preceding claim, comprising an absorbent structure, comprising said superabsorbent material and comprising less than 20%, preferably less than 5%, or less than 1% by weight (of the superabsorbent material) of absorbent cellulose fibers.

12. An absorbent article as in any preceding claim, comprising an absorbent structure, that contains zones comprising said superabsorbent material, and zones comprising a second, different superabsorbent material.

13. An absorbent article as in any preceding claim, comprising an absorbent structure in the form of a laminate or mixture of said superabsorbent material and an adhesive material or thermoplastic material.

14. An absorbent article as in any preceding claim whereby said superabsorbent material has a SFC of at least 30 cm$^3$s10$^{-7}$/g.

15. A process for making an absorbent article comprising a superabsorbent material that comprises superabsorbent polymers and a semiconductor material with radiation-induced hydrophilicity, said process comprising the step of:

a) combining superabsorbent polymers and a material with radiation-induced hydrophilicity; or
b) combining superabsorbent polymers and a material with radiation-inducible hydrophilicity and submitting the resulting mixture to radiation suitable to induce said hydrophilicity, to obtain said superabsorbent material.

16. A process as in claim 15 whereby the material with radiation-induced or -inducible hydrophilicity is applied in the form of a dispersion of particles of said material in a liquid, and whereby said dispersion is applied to the surface of the superabsorbent polymers, preferably in a fluidized bed reactor.

17. A process as in claim 15 where the material with radiation-induced or -inducible hydrophilicity and the superabsorbent polymers are mixed as a dry blend.


**Patentansprüche**

1. Absorptionsartikel, umfassend ein Superabsorptionsmaterial, umfassend Superabsorber-Polymere und ein Halbleitermaterial mit strahlungsinduzierter Hydrophilie, erhältlich durch

a) Kombinieren der Superabsorber-Polymere mit dem Material mit strahlungsinduzierter Hydrophilie; oder
b) Kombinieren der Superabsorber-Polymere mit einem Material mit strahlungsinduzierbarer Hydrophilie, um eine Mischung zu erhalten, und Aussetzen der Mischung gegenüber Strahlung, die geeignet ist, um die Hydrophilie zu induzieren.

2. Absorptionsartikel nach Anspruch 1, wobei das Superabsorptionsmaterial ein Flüssigkeitsanstiegshöhenverhältnis (Fluid Rise Height Ratio, FRH ratio) von mindestens 1,10 besitzt.

3. Absorptionsartikel nach Anspruch 2, wobei das FRH-Verhältnis 1,25 oder mehr oder vorzugsweise 1,40 oder mehr beträgt.

4. Absorptionsartikel nach einem der Ansprüche 2 oder 3, wobei die Höhenzunahme von n-Heptan (Rise in Height of n-heptane, RHH) von 5 cm bis 19 cm beträgt.

5. Absorptionsartikel nach einem der vorstehenden Ansprüche, wobei das Material mit strahlungsinduzierter Hydrophilie in einer Konzentration von 0,01 Gew.-% bis 2 Gew.-% der Mischung von Superabsorptionsmaterial vorhanden ist.

6. Absorptionsartikel nach einem der vorstehenden Ansprüche, wobei das Superabsorptionsmaterial einen Feuchtigkeitsgehalt von weniger als 2 Gew.-%, vorzugsweise weniger als 1 Gew.-% besitzt.

7. Artikel nach Anspruch 1, wobei die Strahlung elektromagnetische Strahlung mit einer Wellenlänge von 200 nm bis 480 nm ist.

8. Artikel nach Anspruch 1, wobei das Material mit strahlungsinduzierter Hydrophilie $TiO_2$, $SnO_2$, $ZnO$, $WO_3$, $V_2O_5$ oder eine Mischung davon umfasst, vorzugsweise wenigstens $TiO_2$.

9. Absorptionsartikel nach Anspruch 1, wobei das Material mit strahlungsinduzierter Hydrophilie eine Beschichtung auf der Oberfläche der Superabserber-Polymere bildet.

10. Absorptionsartikel nach einem der vorstehenden Ansprüche, umfassend eine Absorptionsstruktur mit einem oder mehreren Bereichen, die das Superabsorptionsmaterial umfassen und eine Basiskapazität von mehr als 6,5 $1/m^2$ und/ oder eine Absorptionseffizienz von mindestens 3 $ml/cm^3$ aufweisen.

11. Absorptionsartikel nach einem der vorstehenden Ansprüche, umfassend eine Absorptionsstruktur, umfassend das Superabsorptionsmaterial und umfassend weniger als 20 Gew.-%, vorzugsweise weniger als 5 Gew.-% oder weniger als 1 Gew.-% (des Superabsorptionsmaterials) absorbierende Cellulosefasern.

12. Absorptionsartikel nach einem der vorstehenden Ansprüche, umfassend eine Absorptionsstruktur, die Bereiche enthält, die das Superabsorptionsmaterial umfassen, und Bereiche, die ein zweites, anderes Superabsorptionsmaterial umfassen.

13. Absorptionsartikel nach einem der vorstehenden Ansprüche, umfassend eine Absorptionsstruktur in Form eines Laminats oder einer Mischung des Superabsorptionsmaterials und eines Haftmittelmaterials oder Thermoplastmaterials.

14. Absorptionsartikel nach einem der vorstehenden Ansprüche, wobei das Superabsorptionsmaterial eine SFC von mindestens 30 $cm^3 s 10^{-7}/g$ aufweist.

15. Verfahren zum Herstellen eines Absorptionsartikels, umfassend ein Superabsorptionsmaterial, das Superabsorber-Polymere und ein Halbleitermaterial mit strahlungsinduzierter Hydrophilie umfasst, wobei das Verfahren die folgenden Schritte umfasst:

a) Kombinieren von Superabsorbet-Polymeren mit einem Material mit strahlungsinduzierter Hydrophilie; oder
b) Kombinieren von Superabsorber-Polymeren mit einem Material mit strahlungsinduzierbarer Hydrophilie und Aussetzen der resultierenden Mischung gegenüber Strahlung, die geeignet ist, um die Hydrophilie zu induzieren, um das Superabsorptionsmaterial zu erhalten.

16. Verfahren nach Anspruch 15, wobei das Material mit strahlungsinduzierter oder -induzierbarer Hydrophilie in Form einer Dispersion von Teilchen des Materials in einer Flüssigkeit aufgetragen wird, und wobei die Dispersion auf die Oberfläche der Superabsorber-Polymere aufgetragen wird, vorzugsweise in einem Fließbettreaktor.

17. Verfahren nach Anspruch 15, wobei das Material mit strahlungsinduzierter oder -induzierbarer Hydrophilie und die Superabsorber-Polymere als Trockenmischung gemischt werden.

**Revendications**

1. Article absorbant comprenant un matériau superabsorbant, comprenant des polymères superabsorbant et un matériau semi-conducteur doté d'une hydrophilie induite par un rayonnement, pouvant être obtenu par

a) combinaison desdits polymères superabsorbants et dudit matériau doté d'une hydrophilie induite par un rayonnement ; ou
b) combinaison desdits polymères superabsorbants et un matériau doté d'une hydrophilie pouvant être induite par un rayonnement, de façon à obtenir un mélange et soumission dudit mélange à un rayonnement approprié pour induire ladite hydrophilie.

2. Article absorbant selon la revendication 1, selon lequel ledit matériau superabsorbant a un rapport de hauteur de montée de fluide (rapport FRH) d'au moins 1,10.

**3.** Article absorbant selon la revendication 2, selon lequel le rapport FRH est 1,25 ou plus, ou de préférence 1,40 ou plus.

**4.** Article absorbant selon l'une quelconque des revendications 2 ou 3, selon lequel la hauteur de montée du n-heptane (RHH) va de 5 cm à 19 cm.

**5.** Article absorbant selon l'une quelconque des revendications précédentes, selon lequel ledit matériau doté d'une hydrophilie induite par un rayonnement est présent à un taux de 0,01 % à 2 % en poids du mélange du matériau superabsorbant.

**6.** Article absorbant selon l'une quelconque des revendications précédentes, selon lequel ledit matériau superabsorbant a une teneur en humidité inférieure à 2 % en poids, de préférence inférieure à 1 % en poids.

**7.** Article selon la revendication 1, selon lequel ledit rayonnement est un rayonnement électromagnétique d'une longueur d'onde allant de 200 nm à 480 nm.

**8.** Article selon la revendication 1, selon lequel ledit matériau doté d'une hydrophilie induite par un rayonnement comprend $TiO_2$, $SnO_2$, $ZnO$, $WO_3$, $V_2O_5$, ou un mélange de ceux-ci, de préférence au moins $TiO_2$.

**9.** Article absorbant selon la revendication 1, selon lequel le matériau doté d'une hydrophilie induite par un rayonnement forme un revêtement sur la surface des polymères superabsorbants.

**10.** Article absorbant selon l'une quelconque des revendications précédentes, comprenant une structure absorbante avec une ou plusieurs régions comprenant ledit matériau superabsorbant et ayant une capacité de base de plus de 6,5 $L/m^2$ et/ou une efficacité d'absorption d'au moins 3 $mL/cm^3$.

**11.** Article absorbant selon l'une quelconque des revendications précédentes, comprenant une structure absorbante, comprenant ledit matériau superabsorbant et comprenant moins de 20 %, de préférence moins de 5 %, ou moins de 1 % en poids (du matériau superabsorbant) de fibres de cellulose absorbantes.

**12.** Article absorbant selon l'une quelconque des revendications précédentes, comprenant une structure absorbante, qui contient des zones comprenant ledit matériau superabsorbant, et des zones comprenant un deuxième matériau superabsorbant différent.

**13.** Article absorbant selon l'une quelconque des revendications précédentes, comprenant une structure absorbante sous la forme d'un stratifié ou mélange dudit matériau superabsorbant et un matériau adhésif ou matériau thermoplastique.

**14.** Article absorbant selon l'une quelconque des revendications précédentes, selon lequel ledit matériau superabsorbant a une conductivité en flux salin d'au moins 30 $cm^3s10^{-7}/g$.

**15.** Procédé de fabrication d'un article absorbant comprenant un matériau superabsorbant qui comprend des polymères superabsorbants et un matériau semi-conducteur doté d'une hydrophilie induite par un rayonnement, ledit procédé comprenant l'étape consistant a :

   a) combiner des polymères superabsorbants et un matériau doté d'une hydrophilie induite par un rayonnement ; ou
   b) combiner des polymères superabsorbants et un matériau doté d'une hydrophilie pouvant être induite par un rayonnement et soumettre le mélange résultant à un rayonnement approprié pour induire ladite hydrophilie, de façon à obtenir ledit matériau superabsorbant.

**16.** Procédé selon la revendication 15, selon lequel le matériau doté d'une hydrophilie induite ou pouvant être induite par un rayonnement est appliqué sous la forme d'une dispersion de particules dudit matériau dans un liquide, et selon lequel ladite dispersion est appliquée à la surface des polymères superabsorbants, de préférence dans un réacteur à lit fluidisé.

**17.** Procédé selon la revendication 15, où le matériau doté d'une hydrophilie induite ou pouvant être induite par un rayonnement et les polymères superabsorbants sont mélangés en tant que mélange sec.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5419956 A
- GB 2082614 A
- EP 1669394 A
- EP 20287970 A
- US 5244934 A
- US 20040033270 A
- WO 02083193 A
- EP 1498543 A
- EP 0691133 A
- US 5599335 A
- US 5562646 A
- US 5669894 A
- US 4076663 A, Masuda
- US 4062817 A, Westerman
- US 3661875 A
- US 4093776 A
- US 4666983 A
- US 4734478 A

- US 32649 A, Brandt
- US 4625001 A, Tsubakimoto
- US 5140076 A, Harada
- US 6391451 B
- US 6239230 B, Mitchell
- US 6150469 A, Harada
- US 4541871 A, Obayashi
- WO 9216565 A, Stanley
- WO 9008789 A, Tai
- WO 9305080 A, Stanley
- US 4824901 A, Alexander
- US 4789861 A, Johnson
- US 4587308 A, Makita
- US 5164459 A, Kimura
- DE 4020780, Dahmen
- US 6376618 B1
- EP 509708 A, Gartner